# EUROPÄISCHE PATENTANMELDUNG

(11) **EP 4 353 149 A1**
(43) Veröffentlichungstag der Anmeldung: **17.04.2024**
(21) Anmeldenummer: 22200492.1
(22) Anmeldetag: 10.10.2022
(51) Int. Cl.: A61B 5/08, A61B 5/091, A61B 5/00, A61B 5/0536

(54) **VERFAHREN UND VORRICHTUNG ZUR BESTIMMUNG DER LUNGENVENTILATION**

(71) Anmelder: Technische Hochschule Mittelhessen, 35390 Giessen (DE); Justus-Liebig-Universität Gießen, 35390 Gießen (DE)
(72) Erfinder: Sohrabi, Keywan, 35578 Wetzlar (DE); Groß, Volker, 35435 Wettenberg (DE); Sander, Michael, 35415 Pohlheim (DE); Stenger, Manuel, 35390 Gießen (DE); Fischer, Patrick, 35394 Giessen (DE); Jung, Tibor Nikolas, 35794 Mengerskirchen (DE)
(74) Vertreter: Stumpf, Peter

(57) **Zusammenfassung**

Die Erfindung betrifft ein Computer implementiertes Verfahren zur Bestimmung der Stärke und des räumlichen Ausmaßes der Lungenventilation einer Person (P1) umfassend wenigstens die folgenden Schritte:
Ia. Bereitstellen von Akustik-Messdaten (A1) von mehreren Stellen der Lunge der Person (P1), wobei das Bereitstellen erst nach Abschluss der Untersuchung, nachdem die Messung der Person (P1) abschlossen ist, beginnt.
Ib. Bereitstellen von Messdaten der elektrischen Impedanz (11) von mehreren Stellen der Lunge der Person (P1), wobei das Bereitstellen erst nach Abschluss der Untersuchung, nachdem die Messung der Person (P1) abschlossen ist, beginnt.
II. Übergabe der Messdaten (A1, 11) an ein trainiertes Künstliches Neuronales Netz (KNN)
III. Vorverarbeiten der Messdaten (A1, 11) in vorverarbeitete Messdaten (A1*, 11*) durch das Künstliche Neuronale Netz (KNN)
IV. Anwendung des trainierten Künstlichen Neuronalen Netzes (KNN) auf die vorverarbeiteten Messdaten (A1*, I1*) zur Bestimmung der Stärke und des räumlichen Ausmaßes der Lungenventilation der Person (P1) durch das KNN, wobei die Werte der Lungenventilation als Ergebnisdaten (D2) erzeugt werden.

## Beschreibung

Die vorliegende Erfindung betrifft ein Verfahren und eine Vorrichtung zur nichtinvasiven Bestimmung der Lungenventilation.

### Beschreibung und Einleitung des allgemeinen Gebietes der Erfindung

Die Lungenventilation, kurz auch als Ventilation bezeichnet, ist eine entscheidende Voraussetzung für einen effektiven Gasaustausch zwischen Lunge und Blut. Sie sorgt dafür, dass in den Lungenbläschen Sauerstoff (O₂) ins Blut übertreten und CO₂ abgeatmet werden kann. Die Bestimmung der Lungenventilation ist bei vielen Erkrankungen der Lunge für Diagnostik, Therapieplanung und Therapieüberwachung außerordentlich wichtig.

Die Elektrische Impedanztomographie (EIT) ist ein nichtinvasives bildgebendes Verfahren, das auf Messungen elektrischer Leitfähigkeiten im menschlichen Körper basiert und ein strahlungsfreies Monitoring der regionalen Lungenfunktion ermöglicht. Das Verfahren ermöglicht in einzelnen Schnittbildern der Lunge, die Ausbreitung des Tidalvolumens der Lunge darzustellen.

### Stand der Technik

Es gibt verschiedene konventionelle Verfahren und Vorrichtungen um die allgemeine Ventilation der Lunge zu messen. Bestehende Geräte weisen z.B. jedoch nur einen Elektrodenring auf, so dass die Bestimmung der Lungenventilation zwar pro Schichtbild erfolgt, aber keine dreidimensionale und keine dynamische Erfassung der gesamten Lunge möglich ist.

Die bekannten Verfahren können die Ventilation nicht ortsaufgelöst und nicht dynamisch abbilden.

WO20070088539A2 beschreibt ein Verfahren und eine Vorrichtung zur Bestimmung der Lungenventilation aus verschiedenen Messparametern. Messparameter sind dabei EKG Signal und Beschleunigungsmesswerte des Thorax, sowie der O₂-Partialdruck und der CO₂- Partialdruck der ausgeatmeten Luft. DE202004001776U1 offenbart ein akustisches Verfahren zur Bestimmung der Lungenventilation. Hierbei wird mittels Ultraschallmessung erfasst, welche Regionen der Lunge belüftet sind. Dieses Verfahren ist allerdings darauf limitiert, bereits kollabierte Stellen der Lunge zu detektieren. Es liefert keine ortsaufgelöste dynamische Aussage über die Qualität der Lungenventilation.

Die Nutzung der elektrischen Impedanztomographie (EIT) zur Bestimmung der Lungenventilation ist grundsätzlich bekannt.

Die elektrische Impedanztomographie (EIT) ist ein medizinisches Bildgebungsverfahren, das die Änderung des elektrischen Wechselstromwiderstandes (Impedanz) von Körpergewebe für die Bildgebung heranzieht.

EP3628221A1 offenbart ein Verfahren und eine Vorrichtung zur Bestimmung der Lungenventilation indem die Werte der elektrischen Impedanz an verschiedenen Stellen des Thoraxes gemessen und daraus die Lungenventilation bestimmt wird. Dies wird zur Steuerung einer Beatmungsvorrichtung genutzt. DE102013213526A1 offenbart eine Vorrichtung und ein Verfahren, um mittels elektrischer Impedanztomographie (EIT) die allgemeine Lungenventilation zu bestimmen. Dieses Verfahren bestimmt die Lungenventilation direkt aus der Impedanz zwischen Elektroden auf der Oberfläche des Thorax. US 6790183A offenbart ein Verfahren, in dem mittels Sound-analyse der Atemgeräusche zweidimensionale Bilder der Verteilung der Ventilation erzeugt werden. Diese Bilder stehen senkrecht zur Schnittebene in der Frontalebene zur Verfügung.

Durch die immer besser werdende Messtechnik und Software konnten große Fortschritte bei der Entwicklung von EIT-Systemen erzielt werden. Jedoch gibt es bisher keine dreidimensionale ortsaugelöste Darstellung und keine dynamische Darstellung der Ventilation. Es wird nur die Verteilung der Luft, nicht aber die Bewegung und damit kein Fluss erfasst. Bisher können nur einzelne, senkrechte Schnittansichten der Verteilung von Luft und Gewebe ausgewertet werden.

Die Kombination aus EIT und Soundanalyse existiert bisher noch nicht.

Bei vielen Erkrankungen der Lunge ist jedoch eine dreidimensionale und ortsaufgelöste Bestimmung der Lungenventilation von großem Vorteil, um eine verbesserte Diagnostik, Therapieplanung und Therapieüberwachung zu ermöglichen.

Es besteht der Bedarf an einem verbesserten Verfahren zur Bestimmung der Lungenventilation mit dem eine dreidimensionale und ortsaufgelöste Bestimmung der Lungenventilation möglich ist sowie ein eine Vorrichtung mit der das Verfahren durchgeführt werden kann.

### Aufgabe

Aufgabe der vorliegenden Erfindung ist es, die Nachteile im Stand der Technik zu beseitigen und ein verbessertes Verfahren zur Bestimmung der Lungenventilation bereitzustellen, mit dem eine dreidimensionale und ortsaufgelöste Bestimmung der Lungenventilation möglich ist sowie ein eine Vorrichtung bereitzustellen, mit der das Verfahren durchgeführt werden kann.

### Lösung der Aufgabe

Die Lösung dieser Aufgaben ergibt sich aus den Merkmalen des Hauptanspruchs. Weiterhin sind vorteilhafte Ausgestaltungen und Weiterbildungen der Erfindung den Unteransprüchen entnehmbar.

Die Erfindung hat den Vorteil, dass eine zuverlässige dreidimensionale und ortsaufgelöste Bestimmung der Lungenventilation möglich ist, die bei vielen Erkrankungen der Lunge eine sichere Diagnostik, eine gute Therapieplanung und Therapieüberwachung gewährleitet, die den Patienten nicht beeinträchtigt. Zudem wird eine einfache Vorrichtung bereitgestellt, mit der das Verfahren sehr gut durchgeführt werden kann.

Die Erfindung umfasst eine Kombination aus EIT und akustische Soundanalyse.

Die Elektrische Impedanztomographie (EIT) ist ein nichtinvasives bildgebendes Verfahren, das auf Messungen elektrischer Leitfähigkeiten im menschlichen Körper basiert. Die elektrische Leitfähigkeit biologischer Gewebe unterscheidet sich je nach Beschaffenheit (*absolute EIT*) und/oder funktionellem Zustand (*funktionelle oderrelative EIT*) stark. Dies wird bei der EIT genutzt, indem Oberflächenelektroden um eine bestimmte Körperregion an der Haut befestigt werden und zwischen jeweils zwei (meist benachbarten) Elektroden höherfrequente Wechselströme (10 - 100 kHz) mit niedriger Amplitude im einstelligen Milliampere-Bereich fließen. Diese breiten sich dreidimensional im Körper aus und werden von den übrigen, meist zirkulär neuartig in mehreren Schichten um die Untersuchungsebene angeordneten Elektroden gemessen. Dieser Vorgang wird dann beispielsweise vom nächstgelegenen Elektrodenpaar wiederholt, bis eine vollständige Umrundung analog einem kompletten Messzyklus erfolgt ist. Die registrierten Daten eines solchen Messzyklus können digital zu einem Bild in einem sogenannten Tomogramm weiterverarbeitet werden.

Wesentliche Vorteile gegenüber anderen bildgebenden Verfahren, wie der Magnetresonanztomographie oder Computertomographie, bietet die elektrische Impedanztomographie durch eine nicht invasive Anwendung und dem vergleichsweisen geringen apparativen und finanziellen Aufwand. Zudem ist durch die echtzeitfähige und personalunabhängige Ermittlung physiologischer Größen ein kontinuierliches Monitoring (Langzeit-Monitoring) möglich. Dies ist z.B. für die Therapieüberwachung besonders relevant. Die Messungen können aufgrund der wenig aufwändigen Inbetriebnahme und Anwendung stationär oder mobil erfolgen und stellen keine massive physische oder psychische Belastung des Patienten dar.

Die Erfindung betrifft ein verbessertes Verfahren zur Bestimmung der Lungenventilation auf Basis der elektrischen Impedanztomographie (EIT) in Kombination mit einer Soundanalyse, insbesondere der elektronisch erfassten Akustik-Messungen am Thorax. Die Auswertung erfolgt dabei über ein künstliches neuronales Netz. Die Notwendigkeit der Verwendung eines künstlichen neuronalen Netzes ergibt sich aus der Verknüpfung der verschiedenen Messdaten.

Akustische Daten der Lunge werden z.B. mittels Abhören (Auskultation) des Thorax mittels Stethoskop durchgeführt. Die klinische Interpretation dieser Auskultationsbefunde, ist jedoch sehr stark von der akustischen Sensibilität des jeweiligen Untersuchers abhängig. Die Auskultation mit dem Stethoskop bietet leider keine Möglichkeit, das Gehörte aufzuzeichnen und objektiv zu vergleichen. Die automatische Aufzeichnung der Atmungsgeräusche erfolgt daher über piezoelektrische Kontaktsensoren oder mit Mikrofonen, die an üblichen Standard-Auskultationspunkten am Thorax befestigt werden. Die Daten werden einzeln aufgezeichnet und bewertet. Eine Interpretation der Lautstärke ermöglicht so z. B. die Bewertung der lokalen Belüftung.

Künstliche Neuronale Netze, auch Künstliche Netzwerke, kurz: KNN (englisch artificial neural network, ANN), sind Netze aus künstlichen Neuronen. Künstliche Neuronale Netze basieren auf der Vernetzung vieler einzelner, meist in Schichten angeordneter, künstlicher Neuronen. Die Intensität der Verbindungen zwischen den Neuronen wird durch sogenannte Gewichtungen/Gewichte gesteuert. Die Topologie sowie die steuernden Parameter eines Netzes werden in Abhängigkeit der Problemstellung gewählt. In einer Trainingsphase werden KNN anhand von Eingangsdaten (Eingangssignal), für die erwartete Ergebnisse (Soll-Signal) vorliegen, durch Vergleich dieser mit den errechneten Ergebnissen (Ergebnis-Signal) angelernt. Die Differenz von Ergebnis- und Soll-Signal wird als Loss bezeichnet. Der Loss zeigt an, wie gut das erwartete und das tatsächliche Ergebnis übereinstimmen. Um die Qualität der Verarbeitung zu verbessern, muss dieser Loss für zukünftige Durchläufe berücksichtigt werden. Diese Anpassung der Verarbeitung bezeichnet man als Backpropagation bzw. Gewichts-Anpassung. Hierbei werden die Gewichte zwischen den Neuronen derart justiert, dass sie zukünftig dem gewünschten Ergebnis näherkommen. Basierend darauf kann die Lernfähigkeit von KNNs als Fähigkeit zum Vergleich eines bestimmten Ergebnisses und eines erwarteten Ergebnisses, gefolgt von einer Anpassung entsprechend der Differenz zwischen diesen Werten, definiert werden.

KNNs sind dadurch in der Lage, mithilfe eines iterativen Trainingsprozesses komplizierte nichtlineare Funktionen zu erlernen.

Ein weit verbreitetes Prinzip für das Training von KNN stellt das überwachte Lernen dar. Hierbei wird das Neuronale Netz mit einer Menge von Tupeln aus zusammengehörenden Eingangs- (x) und Ausgangssignalen (y) mithilfe des zuvor beschriebenen Prinzips trainiert und iterativ angepasst. Die Menge von Tupeln (x, y), die für das Training genutzt werden, bezeichnet man als Trainingsdaten (D_{training}). Die Funktion des Netzes auf ihm unbekannte Daten wird mithilfe von Validierungsdaten (D_{validation}) ermittelt. Diese Menge von Tupeln (x, y) enthält Daten, mit denen das Netzt nicht trainiert wurde. Es gilt D_{training} ∩ D_{validation} = Ø.

### Zum Training des neuronalen Netzes:

Aus dem Vergleich der aus Akustik-Trainingsdaten TA1 und der aus Trainingsmessdaten der elektrischen Impedanz TI1 berechneten Ist-Werte mit den Vergleichsdaten D1 der Trainingsdaten, werden die im Künstlichen Neuronalen Netz enthaltenen Gewichte angepasst. Dieser iterative Prozess endet mit einem autark anwendbaren neuronalen Netz *N*.

Um das Lernen zu beschleunigen und die Genauigkeit des Segmentierungsmodells zu verbessern, soll der Vorteil des Transferlernens genutzt werden. Der Hauptvorteil der aktuellen Methode gegenüber den merkmalsbasierten Ansätzen besteht darin, dass die Merkmalsextraktion und -auswahl durch das Netzwerk erfolgt, was zu einem ausgeklügelten Merkmalskombinationssatz führt. Diese können auf der Grundlage des angepassten Datensatzes auf einen bestimmten Anwendungsfall angewendet werden. Das Modell soll mit vortrainierten Gewichten, dem ILSVRC Image-Net-Datensatz25 initialisiert werden. Das Modell soll mit dem Adam-Optimierer unter Verwendung von "dice loss-Verfahren" mit angepassten Klassengewichten trainiert werden.

Hierdurch wird das Netz *N* zur Mustererkennung befähigt. Bei der Nutzung von *N* zur Vorhersage erfolgt daher kein Zugriff auf konkrete Datensätze der Trainingsdaten, sondern ausschließlich auf die erlernten Gewichte. Die Vorhersage erfolgt damit nur auf Basis dieser durch das Training erlernten Gewichte.

### Trainingsphase

Vor der eigentlichen Durchführung des erfindungsgemäßen Verfahrens muss das KNN eine Trainingsphase als einen Schritt 0 durchlaufen.

Dieser Schritt 0 umfasst folgende Teilschritte:
**0a.** Empfangen der Akustik-Trainingsdaten TA1 und Trainingsmessdaten der elektrischen Impedanz TI1.
**0b.** Vorverarbeitung der Akustik-Trainingsdaten TA1 zu den Akustik-Trainingsdaten TA1* und Vorverarbeitung der Trainingsmessdaten der elektrischen Impedanz TI1 zu den Trainingsmessdaten der elektrischen Impedanz TI1*, Übergabe dieser vorverarbeiteten Trainingsmessdaten TA1* und TI1* an das Künstliche Neuronale Netz *N*.

### Vorverarbeitung:

Um für das Training des Netztes qualitativ hochwertige Daten zu nutzen, müssen die verwendeten Daten wie TA1, TI1 zu vorverarbeiten Messdaten TA1 *, TI1* aufbereitet werden. Dies wird auch als Vorverarbeitung bezeichnet. Hierbei besteht die Vorverarbeitung der klinischen Daten D1 aus einer semi-automatisierten Segmentierung von Thorax-CT Aufnahmen. Die segmentierten Lungenareale dienen als Referenz D1* für das Künstliche Neuronale Netz. Dazu muss eine sogenannte Daten-Pipeline eingerichtet werden. Die Daten-Pipeline dient dazu, die heterogen gespeicherten Daten in bereinigte Datensätze zu überführen, die problemlos im weiteren Verfahren zu verwenden sind. Die Vorverarbeitung berücksichtigt geeignete Maßnahmen zur Anreicherung (Data Augmentation) der Daten.

Die Vorverarbeitung in der Trainingsphase umfasst folgende Schritte:

### a. Extraktion

Die Akustik-Messdaten A1 und die Messdaten der elektrischen Impedanz I1 der einzelnen Patienten werden aus den Rohdaten extrahiert. Die Messdaten liegen hierbei in einem binären Dateiformat vor. Die Referenzdaten D1 liegen z.B. im DICOM-Format vor, wobei es herstellerspezifische Unterschiede in der Formatierung dieser Daten gibt. Die vorverarbeiteten Referenzdaten D1* werden mit Hilfe semiautomatischer Segmentierung erstellt und haben ebenfalls das DICOM Format. Anschließend werden die Mess-Daten und die Diagnose-Informationen je eines Patienten zu einem Tupel (x, y) verknüpft.

### b. Auswahl

In diesem Schritt werden nicht verwendbare Datensätze von der weiteren Verarbeitung ausgeschlossen (z. B. unvollständige Datensätze, die nicht imputiert werden können). Es sollen nur vollständige Datensätze für das Training genutzt werden, um das Erlernen von Korrelationen zu optimieren.

### c. Bereinigung

Die Mess-Aufzeichnungen TA1, werden beschnitten, um technische Artefakte z.B. am Beginn und/oder am Ende zu beseitigen.

### d. Augmentierung

Um die für das Training verwendbare Datenmenge zu erhöhen und dadurch die Performance des Netzes zu steigern, werden eindeutige Teilausschnitte aus jeder Messwert-Aufnahme extrahiert. Hierbei wird ein Fenster fixer Größe Schritt für Schritt über die Aufnahme bewegt, was jeweils eine neue Aufnahme generiert. Dies ermöglicht das Erhöhen der Datenmenge um bspw. den Faktor 100. Weitere Methoden der Augmentierung beinhalten Verschiebung, Rotation und Weitere und dienen zur Erweiterung des Datensatzes.

### e. Formatierung

Um hochfrequentes Rauschen und die Größe der Mess-Aufnahmen zu reduzieren, wird für jeden Datensatz ein Down-Sampling durchgeführt. Dies reduziert durch ein Average-Pooling-basiertes Verfahren Details der Aufnahme, erhält jedoch deren signifikante Muster. Hierdurch lässt sich die Datengröße um bspw. den Faktor 4 reduzieren.

### f. Speichern

Die Daten werden in gleichgroße Gruppen aufgeteilt. Abhängig vom gewünschten Verhältnis zwischen Trainingsdaten und Validierungsdaten (bevorzugt ist hierbei ein Verhältnis von 80% zu 20%) wird eine bestimmte Anzahl von Gruppen (z.B. 6) für das Training verwendet, der Rest für die Validierung. Diese Konstellation aus Trainings- und Validierungssplit wird je als eine Datensatz-Variante gespeichert. Dieser Vorgang wird so lange wiederholt, bis entweder genügend Varianten angelegt sind oder alle Kombinationen von Gruppen im Training/Validierung aufgetreten sind. Dies ermöglicht das Training identisch aufgebauter Modelle mit unterschiedlichen Daten. In Kombination liefern diese Modelle (z.B. 6) eine höhere Performance auf unbekannte Daten als ein einzelnes Modell aus dieser Menge. Man bezeichnet dieses Verfahren als Ensembling.
**0c**. Ermittlung von der Stärke und des räumlichen Ausmaßes der Lungenbelüftung durch das Künstliche Neuronale Netz *KNN.*
**0d**. Eingabe der Vergleichsdaten D1.

Die Vergleichsdaten D1 dienen als Referenzdaten zum Training des KNN.
**0e**. Iterativer Soll-Ist-Vergleich der ermittelten Wahrscheinlichkeiten mit den Vergleichsdaten D1 mit anschließendem Anpassen der Gewichte G im Künstlichen Neuronalen Netz *KNN.*
**0f**. Fertigstellen des trainierten Künstlichen Neuronalen Netzes *N* mit den angepassten Gewichten G.

Nach der Trainingsphase wird das erfindungsgemäße Verfahren durchgeführt.

### Bestimmung der Lungenventilation

Die in einem vorangegangenen Schritt (der nicht Teil des erfindungsgemäßen Verfahrens ist) erhobenen Akustik-Trainingsdaten TA1 von verschiedenen Stellen des Thoraxes und die Trainingsmessdaten der elektrischen Impedanz werden als Eingangssignale des erfindungsgemäßen Verfahrens verwendet.

Für das Training des neuronalen Netzes werden bevorzugt, Akustik-Trainingsdaten TA1 von verschiedenen Stellen des Thoraxes und Trainingsmessdaten der elektrischen Impedanz TI1, sowie die auf Basis von durch etablierte bildgebende Verfahren z.B. segmentierte CT-Daten erzeugte Vergleichsdaten D1 genutzt. Die Messdaten stellen dabei die Eingangssignale (TA1, TI1, D1) dar, während die Vergleichsdaten D1 die Zielwerte für die Ausgangs-Signale des Netzes bereitstellen. Diese Menge von Tupeln aus (x, y) bilden die Trainingsdaten (D_{training}). Hierbei entspricht ein x einem Tupel aus den Daten (TA1, TI1, D1) und ein y einem Vektor mit der Vergleichsdaten D1. Aus den Eingangssignalen x wird über das KNN ein Ergebnissignal y` ermittelt, was wie zuvor beschrieben mit dem wahren y abgeglichen und zur Berechnung des Loss genutzt wird.

### Erfindungsgemäßes Verfahren:

Das erfindungsgemäße Verfahren zur Bestimmung der Stärke und des räumlichen Ausmaßes der Lungenventilation einer Person P1 umfasst dabei wenigstens die folgenden Schritte:
Ia: Bereitstellen von Akustik-Messdaten A1 von mehreren Stellen der Lunge einer Person (P1), wobei das Bereitstellen erst nach Abschluss der Untersuchung, nachdem die Messung am Patienten abschlossen ist, beginnt.
Ib: Bereitstellen von Messdaten der elektrischen Impedanz I1 von mehreren Stellen der Lunge der Person (P1), wobei das Bereitstellen erst nach Abschluss der Untersuchung, nachdem die Messung am Patienten abschlossen ist, beginnt.
II: Übergabe der Messdaten (A1, 11) an ein Künstliches Neuronales Netz (KNN)
III. Vorverarbeiten der Messdaten (A1, 11) in vorverarbeitete Messdaten (A1*, I1*),
IV: Anwendung des trainierten Künstlichen Neuronalen Netzes *KNN* auf die vorverarbeiteten Messdaten (A1*, 11*) zur Ermittlung von der Stärke und dem räumlichen Ausmaß der Lungenventilation an mehreren Punkten der Lunge der Person (P1), durch das KNN.

Die Reihenfolge der Schritte Ia und Ib ist dabei beliebig wählbar.

Die Durchführung des Verfahrens erfolgt durch eine Vorrichtung zur elektronischen Datenverarbeitung PC, Smartphone, Mac. Es handelt sich um ein computerimplementiertes Verfahren.

Die Frequenz der Akustik-Messdaten A1 und der Messdaten der elektrischen Impedanz I1, welche in Schritt Ia und Ib bereitgestellt werden, umfasst wenigstens einen Bereich von 50 bis 3000 Hz. Dies stellt eine zuverlässige dynamische dreidimensionale Bestimmung der Lungenventilation sicher. Abweichungen von der Stärke und des räumlichen Ausmaßes der Lungenventilation gegenüber einer Vergleichsperson können somit sehr gut und sehr schnell festgestellt werden.

Nach Abschluss des Schnitts IV wird vorzugsweise aus den Ergebnisdaten (D2) eine dreidimensionale Abbildung erzeugt. Dies dient dazu die Stärke und das räumliche Ausmaß der Lungenventilation visuell sichtbar zu machen und Abweichungen darstellen zu können.

Nach der Messwertbereitstellung folgt die Rekonstruktion der Leitwertverteilung aus den vorverarbeiteten Messdaten der elektrischen Impedanz I1*. Hierbei lassen sich aufgrund dessen, dass aus den gemessenen Oberflächenpotentialen die interne Leitwertverteilung wiedergegeben werden soll, zwei Probleme formulieren: Das Vorwärtsproblem und das inverse Problem. Beim sog. Vorwärtsproblem wird nach der Lösung für das Potential im Objektinneren und am Objektrand bei bekannter Leitwertverteilung, Objektgeometrie und Stromeinspeisung gesucht.

In umgekehrter Richtung ist die Leitwertverteilung im Objektinneren unbekannt, bei gegebenen Randwerten für Strom und Spannung sowie der Objektgeometrie.
Im Allgemeinen ist die analytische Bestimmung des Vorwärtsproblems auf einfache Objekt Geometrien beschränkt, sodass die numerische Lösung mittels Finite-Element-Methode (FEM) häufig Anwendung findet. Dieses Verfahren teilt die Fläche innerhalb der Elektrodenebene in eine endliche Anzahl an Dreiecken (Finite Elemente) auf, wobei jedem Dreieck homogene und isotrope bioelektrische Eigenschaften zugewiesen werden. Ziel ist es, die spezifischen Widerstandswerte jedes einzelnen Dreieckes aus den Messdaten zu bestimmen. Der Initialwert für die spezifischen Widerstände ist im einfachsten Fall die homogene Verteilung. Anschließend wird mittels FEM bestimmt, welche Randpotentiale bei allen Stromeinspeisungspaaren auftreten müssten und mit den tatsächlich messtechnisch bestimmten Potentialen verglichen. Aus der Differenzbildung der beiden Spannungswerte wird dann möglichst effizient die nächste Iteration für die spezifischen Widerstände berechnet. Durchaus mathematisch anspruchsvoll und nicht immer eindeutig sind dabei die Interpretationen der Signale bei unbekannter Verteilung der Leitwerte (inverses Problem). Ausgangspunkt sind immer (mehr oder weniger komplexe) theoretische Verteilungs-Modelle. Diese Verteilungsmodelle könnten wie bereits beschrieben zukünftig aber auch aus echten Patientendaten vorab mit anderen Methoden (z.B. CT), in der in der Trainingsphase quasi als individuelle Basis erstellt werden. Dabei wird hierbei z.B. auf segmentierte CT-Daten der Patienten zurückgegriffen. Dies würde die Methode vereinfachen und die Auflösung und Eindeutigkeit der Methode erheblich verbessern.

Die Auskultation stellt in der heutigen Medizin das Standardverfahren zur pulmologischen und kardiologischen Statusbestimmung von Patienten dar. Sie ist eines der ältesten und bewährtesten diagnostischen Verfahren zur Beurteilung der lokalen Lungenbelüftung. Das normale Atemgeräusch ist in einem Frequenzbereich von 75 Hz bis über 2000 Hz angesiedelt. Der Ursprung des normalen Atemgeräusches, welches über den peripheren Lungenabschnitten auskultierbar ist, befindet sich in den Arealen der größeren Atemwege. Genauere Untersuchungen ergaben, dass der genaue Entstehungsort des normalen Atemgeräusches innerhalb der Bronchien liegen muss. Die Lautstärke des normalen Atemgeräusches ist in den verschiedenen Phasen des Atemzyklus in den belüfteten Lungenarealen unterschiedlich. Das lauteste Geräusch entsteht zu Beginn der Inspiration in den oberen Lungenarealen. Bei zunehmenden Atemzugvolumina finden sich in den unteren Lungenarealen ebenfalls große Lautstärke-Amplituden. Diese Geräusche werden mittels Mikrophone oder piezoelektrische Sensoren erfasst, die die passive Vibrationsenergie der Lunge während des Atemzyklus registrieren. Diese als A1 erfassten Daten Können gespeichert und konvertiert werden. Mit ihnen ist es möglich, ein funktionelles, dynamisches Lungenbild zu erstellen. Dadurch können Veränderungen im Luftfluss oder der Gewebezusammensetzung dargestellt werden. Durch die Quantifizierung der Vibrationsverteilung gestatten die Daten A1 eine regionale Bewertung der linken und rechten Lungenbelüftung. Die Erfassung der Messdaten A1 erfolgt z.B. über eine Mess-Weste 110. In textile Schichten sind Sensorarrays integriert (Mikrophone oder piezoelektrische Sensoren. Hierbei detektieren die akustischen Sensoren (piezoelektrisch oder Mikrophone) die Signale in 30 Sekunden Segmenten und leiten sie an einen (Zwischen-) Speicher 90 weiter und können dann in einer Rechnereinheit weiterverarbeitet werden. Das computergestützte Verfahren transformiert die aufgezeichneten akustischen Signale unter Zuhilfenahme unterschiedlicher Bildrekonstruktionsalgorithmen in eine dynamische Darstellung der Lungenventilation. In diesem Zusammenhang ist die Intensität der Atemgeräusche ein direktes Maß für die lokale Lungenbelüftung in der Nähe der Sensorpositionen unter Berücksichtigung der fortgeleiteten Atemgeräusche.

Das erfindungsgemäße Verfahren ermöglicht ein dreidimensionales Erfassen der gesamten Lunge und nicht nur einzelner Schnittebenen. Somit ist eine dynamische dreidimensionale Darstellung der Lungenventilation möglich und Abweichungen in der Stärke und des räumlichen Ausmaßes der Lungenventilation sind leicht erkennbar. Das Verfahren ist nichtinvasiv und benötigt keine radioaktive Strahlung. Es kann durchgeführt werden, um den zeitlichen Verlauf der Lungenventilation z.B. während einer Therapie zu überwachen. Die erfassten Daten werden automatisiert ausgewertet und können auch in Echtzeit dargestellt werden. Dieses Verfahren wird dabei als Software umgesetzt. Das erfindungsgemäße Verfahren wird als App oder als Zusatzmodul zu bisheriger Auswertungssoftware z.B. für Geräte zur elektrischen Impedanztomographie (EIT) bereitgestellt. Der medizinische Nutzen liegt einer dynamischen Ventilationsmessung zur Diagnostik von Lungenerkrankungen und bzw. zur Überwachung von Therapien von Lungenerkrankungen z.B. bei Asthma und be COPD (chronisch obstruktiver Lungenerkrankung). Abweichungen der Stärke und des räumlichen Ausmaßes der Lungenventilation sind rasch erkennbar und eine Therapie kann schnell angepasst werden.

Das Verfahren dient der Visualisierung der dynamischen Lungenventilation sowie die kardialen Aktivitäten. Es ist ein nicht-invasives und strahlungsfreies Verfahren zur Lungen- und Herzdiagnostik. Dank der Möglichkeit des kontinuierlichen Langzeit-Monitoring kann das System sowohl im Klinikumfeld als auch auf dem Homecare-Sektor eingesetzt werden. Aufgrund seiner hohen Mobilität ermöglicht es eine bettseitige Diagnostik am Krankenbett. Neben einer kontinuierlichen Überwachung der Lungenperipherie schafft das mitarbeitsunabhängige System die Möglichkeit sowohl die Belüftung des globalen Lungenstatus als auch die Ventilation spezifischer Lungenregionen zu visualisieren und ermöglicht somit Ärzten entstehende Pathologien frühzeitig zu erkennen. Zum einen kann die durchgeführte Diagnostik nahezu online am Krankenbett bewertet werden, zum anderen zeichnet das System alle akquirierten Signale auf, sodass die Möglichkeit besteht zu einem späteren Zeitpunkt eine Diagnose zu erheben bzw. die Daten erneut bewertet werden können. Durch seine Flexibilität kann das Verfahren bei spontanatmenden, beatmeten und nicht-invasiv beatmeten Patienten angewandt werden.

### Vorrichtung:

Die erfindungsgemäße Vorrichtung 1 zur Bestimmung der Stärke und des räumlichen Ausmaßes der Lungenventilation umfasst wenigstens die folgenden Elemente:

### Eingabeeinheit 10:

Die Eingabeeinheit 10 ist so ausgebildet, dass sie die Messdaten A1, I1 empfangen und an eine Auswertungseinheit 20 weiterleiten kann. Die Messdaten können dabei direkt von Messgeräten, wie z.B. einer Mess-Weste 110 stammen und über einen (Zwischen-) Speicher 90 übermittelt werden. Die Datenübermittlung erfolgt dabei über eine feste Datenleitung z.B. über eine interne Datenleitung, Lokal-Area-Network (LAN) etc. oder drahtlos z.B. über Wireless Lokal-Area-Network (WLAN), Bluetooth etc.

Bei der Eingabeeinheit 10 kann es sich um eine Tastatur oder um eine grafische Benutzeroberfläche oder auch grafische Benutzerschnittstelle (Abk. GUI von englisch graphical user interface) handeln.

### Datenschnittstelle 50

Die Datenschnittstelle 50 ist so ausgebildet, dass sie die Messdaten A1, I1 aus einem externen Datenverarbeitungssystem empfangen und die Ergebnisdaten an ein externes Datenverarbeitungssystem weiterleiten kann. Die Datenübermittlung erfolgt dabei über eine feste Datenleitung z.B. über ein Lokal-Area-Network (LAN) etc. oder drahtlos z.B. über Wireless Lokal-Area-Network (WLAN), Bluetooth etc. Die Datenschnittstelle 50 ist üblicherweise auf den Austausch mit verschiedenen externe Datenverarbeitungssysteme ausgelegt, d.h. gängige Schnittstellen zum Datenaustausch sind z.B. USB, Cardreader, LAN, WLAN, Bluetooth. Die Datenschnittstelle 50 ist so ausgebildet, dass ein Anwender mit einem beliebigen webfähigen Gerät z.B. Tablet, PC, Smartphone im lokalen Netzwerk über Datenschnittstelle 50 auf die Vorrichtung 1 zugreifen kann.

### Auswertungseinheit 20

Die Auswertungseinheit 20 ist so ausgebildet, dass sie die Messdaten A1, I1 von der Eingabeeinheit 10 oder einer Datenschnittstelle 50 empfangen kann. Die Datenübermittlung erfolgt dabei über eine feste Datenleitung z.B. über eine interne Datenleitung, Lokal-Area-Network (LAN) etc. oder drahtlos z.B. über Wireless Lokal-Area-Network (WLAN), Bluetooth etc. Es handelt sich um eine Vorrichtung zur elektronischen Datenverarbeitung wie beispielsweise ein PC, Smartphone oder Mac. Die Auswertungseinheit 20 ist weiterhin so ausgebildet, dass sie aus den Messdaten A1, I1 und aus den mittels Trainingsdaten enthaltenen erlernten Gewichten die Lungenventilation an verschiedenen Punkten der Lunge durch das Künstliche Neuronale Netz berechnen kann. Das Ergebnis dieser Berechnung sind dabei die Ergebnisdaten D2. Als Auswertungseinheit 20 dient dabei ein Gerät zur elektronischen Datenverarbeitung z.B. ein in die Vorrichtung 1 integrierter programmierbarer Mikroprozessor. Die Auswertungseinheit 20 ist weiterhin so ausgebildet, dass sie die Ergebnisdaten D2 an eine Ausgabeeinheit 30 übermitteln kann. Die Datenübermittlung erfolgt dabei über eine feste Datenleitung z.B. über eine interne Datenleitung, Lokal-Area-Network (LAN) etc. oder drahtlos z.B. über Wireless Lokal-Area-Network (WLAN), Bluetooth etc.

Die Auswertungseinheit 20 verwendet ein Künstliches Neuronales Netz zur Mustererkennung. Die Vorrichtung 1 liefert die Werte der Lungenventilation als Ergebnisdaten D2. Diese Daten sagen aus, wie groß die Lungenventilation an einem bestimmten Punkt der Lunge ist, sie gibt Stärke und räumliches Ausmaß der Lungenventilation an. Diese Ergebnisdaten D2 werden über die Ausgabeeinheit 30 in Form visueller und/oder akustischer Ausgaben ausgegeben.

### Ausgabeeinheit 30

Die Ausgabeeinheit 30 ist so ausgebildet, dass sie die Ergebnisdaten D2 der Auswertungseinheit 20 empfangen und ausgeben kann. Die Ausgabe erfolgt optisch, akustisch, haptisch und/oder in Form eines Ausdruckes.

Die Eingabeeinheit 10 und die Ausgabeeinheit 30 der Vorrichtung 1 können dabei z.B. Tasten, LEDs, (Touch) Display und Sprachein-/ausgabe umfassen.

Die Erfassung von Akustik-Messdaten A1 und der Messdaten der elektrischen Impedanz I1 an mehreren Stellen der Lunge einer Person (P1) erfolgt z.B. über eine Mess-Weste 110, die an geeigneten Stellen vorzugsweise im Bereich des Thorax Elektroden 60 und Mikrophone 70 aufweist.

Die Weste ist z.B. eine ärmellose taillenlange Jacke, die den Oberkörper zumindest abschnittsweise bedeckt. Die Weste stellt einen textilen Träger dar, mit Trockenelektroden zur Messung der elektrischen Impedanz, die auf einer Innenseite des textilen Trägers angebracht sind. Wird einer Person diese Weste angelegt, können Messdaten der elektrischen Impedanz I1 an mehreren Stellen der Lunge dieser Person (P1) erfasst werden.

Zusätzlich weist die Mess-Weste 110 im textilen Träger mehrere Mikrophone 70 auf. Wird einer Person diese Weste angelegt, können Akustik-Messdaten A1 an mehreren Stellen der Lunge dieser Person (P1) erfasst werden. In einer Ausführungsform weist die Mess-Weste einen (Zwischen)-Speicher 90 zur Speicherung der Messdaten der elektrischen Impedanz I1 und der Akustik-Messdaten A1 auf. Diese Messdaten werden erst nach Abschluss der Messung vom (Zwischen)-Speicher 90 an die Eingabeeinheit 10 weitergeben. In einer weiteren Ausführungsform weist die Mess-Weste eine Datenschnittselle 50 auf.

### Ausführungsbeispiel

In einer Ausführungsform erfolgt die Bestimmung der Lungenventilation mit einer Mess-Weste 110. Diese wird einer Person am Oberkörper so angelegt, dass Mikrophone 70 und Elektroden 60 einen direkten Hautkontakt zum Thorax oder Teilen des Thorax erhalten und Messdaten der elektrischen Impedanz I1 und der Akustik-Messdaten A1 erhalten werden.

Das Messprinzip der Messdaten I1 beruht auf der Einspeisung eines sinusförmigen Wechselstroms und der Erfassung der elektrischen Spannungen des untersuchten Körperquerschnitts. Über ein Paar, der 16 kreisförmig sowie über mindestens 6 Reihen am Körperquerschnitt positionierten Oberflächenelektroden, wird ein geringer Wechselstrom (5 mA) mit einer Frequenz von 10 kHz - 1 MHz eingekoppelt. Durch die Stromverteilung im leitfähigen Gewebe entstehen Potentialdifferenzen und Äquipotentiallinien. Die übrigen 93 Elektrodenpaare (bei 6 Reihen mit 16 Elektroden pro Schicht), welche nicht an der Stromeinspeisung beteiligt sind, detektieren die resultierenden Spannungen an der Körperoberfläche. Die detektierten Oberflächenspannungen werden von der intrathorakalen Impedanzverteilung bedingt. Vornehmlich hängt der ermittelte Widerstand vom intrapulmonalen Luftgehalt ab. Des Weiteren tragen intrathorakale Fluidverschiebungen und der pulsatile Blutfluss zum Widerstand bei. Nach Aufzeichnung aller Oberflächenspannungen, rotiert die Stromeinspeisung zum benachbarten Elektrodenpaar. Aus den aufgezeichneten Spannungswerten und dem eingekoppelten Strom wird die elektrische Impedanz ermittelt und die Information des Körperinneren als "frame" dargestellt, welcher zur Rekonstruktion des Querschnittbildes dient. Das Verfahren akquiriert die Daten mit einer Frequenz von bis zu 44 Messzyklen/s, wobei ein vollständiger Messzyklus 1.248 (16 Stromeinspeisungen x 13 Oberflächenspannungen x 6 Schichten) Messwerte liefert. Nach dem Reziprozitätstheorem resultiert aus dem Vertauschen der Elektroden zur Spannungsmessung und Stromeinspeisung dieselbe Transferimpedanz.

In einer Ausführungsform weist die Mess-Weste einen Signalwandler 80 auf, der die Messdaten in eine geeignete Form wandelt. In einer weiteren Ausführungsform weist die Mess-Weste weiterhin einen (Zwischen-) Speicher 90 auf zur Speicherung der Messdaten der elektrischen Impedanz I1 und der Akustik-Messdaten A1. Diese Messdaten werden nach Abschluss der Messung vom (Zwischen)-Speicher 90 an die Eingabeeinheit 10 weitergeben oder über die Eingabeeinheit 10 erstmals eingegeben.

Nach der Durchführung des Verfahrens wird in einem Thoraxquerschnitt die regionale Impedanzverteilung ermittelt und diese sowohl mit dem regionalen Lungenvolumen als auch mit der regionalen Ventilation korreliert. Somit ist es möglich die Stärke und das räumliche Ausmaß der Lungenventilation zu bestimmen.

### Abbildungslegenden

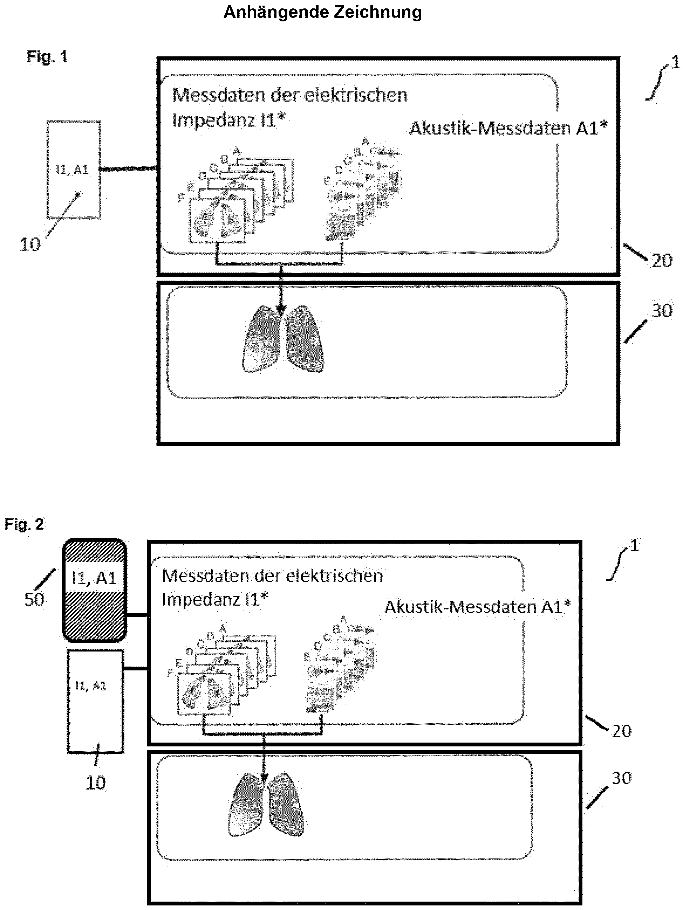

Fig. 1 zeigt den schematischen Aufbau der Vorrichtung 1 in einer Ausführungsform mit einer Eingabeeinheit 10 über die die Daten I1 und A1 eingetragen, erhalten oder empfangen werden.
Fig. 2 zeigt den schematischen Aufbau der Vorrichtung 1 in einer Ausführungsform mit einer Datenschnittstelle 50, die die Daten I1 und A1 erhält oder empfängt. Über die Datenschnittstelle können auch Trainingsdaten erhalten oder empfangen werden.

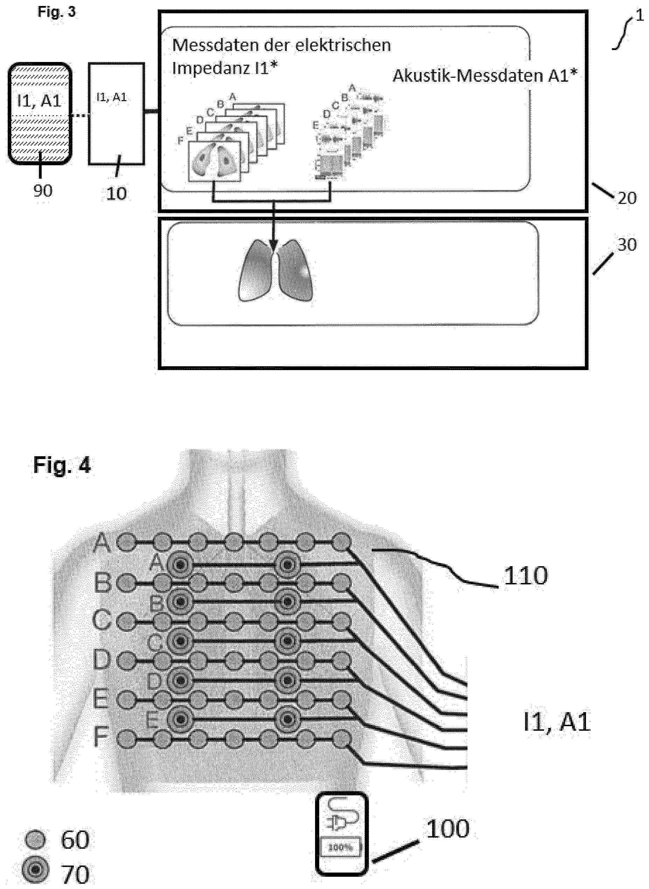

Fig. 3 zeigt den schematischen Aufbau der Vorrichtung 1, wobei die Auswerteeinheit 20 die Daten I1 und A1 vom (Zwischen-) Speicher 90 erhält oder empfängt.
Fig. 4 zeigt die Mess-Weste 110 zur Messung der Daten I1 und A1 in einer ersten Ausführungsform mit Anordnung der Elektroden 60 und Mikrophone 70 sowie einer Stromversorgung 100.

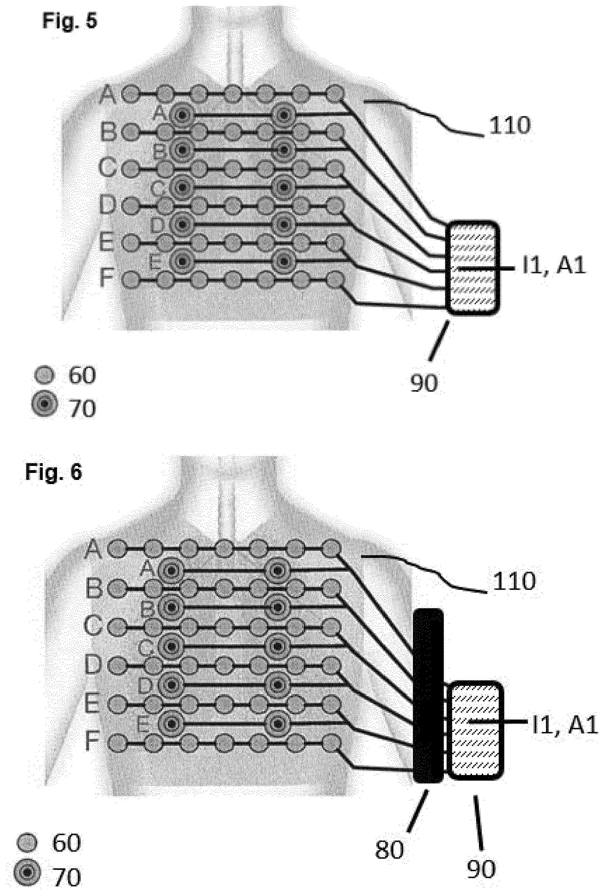

Fig. 5 zeigt die Mess-Weste 110 in einer Ausführungsform mit (Zwischen-) Speicher 90 zur Speicherung der Daten I1 und A1.
Fig. 6 zeigt die Mess-Weste 110 in einer weiteren Ausführungsform mit Signalwandler 80 und (Zwischen-) Speicher 90.

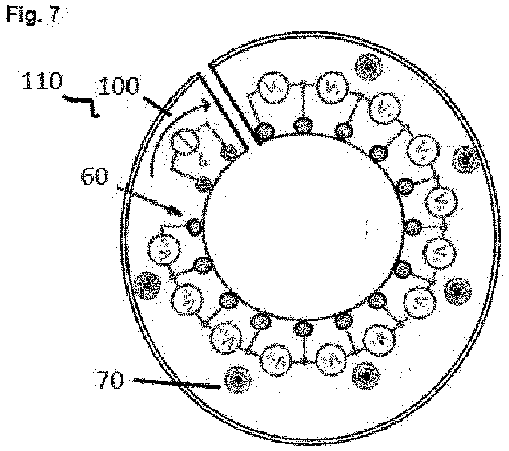

Fig. 7 zeigt einen Querschnitt durch die Mess-Weste 110 mit Anordnung der Elektroden 60 und Mikrophone 70.

### Bezugszeichen

- 1: Vorrichtung
- 10: Eingabeeinheit
- 20: Auswertungseinheit
- 30: Ausgabeeinheit
- 50: Datenschnittstelle
- 60: Elektrode
- 70: Mikrophon
- 80: Signalwandler
- 90: (Zwischen-) Speicher
- 100: Energieversorgung
- 110: Mess-Weste
- TA1: Akustik-Trainingsdaten Trainingsmessdaten der elektrischen Impedanz
- A1: Akustik-Messdaten
- I1: Messdaten der elektrischen Impedanz
- D1: Vergleichsdaten
- D2: Ergebnisdaten
- N: trainiertes Neuronales Netz (KNN)

## Patentansprüche

1. Computer implementiertes Verfahren zur Bestimmung der Stärke und des räumlichen Ausmaßes der Lungenventilation einer Person (P1) umfassend wenigstens die folgenden Schritte:
Ia. Bereitstellen von Akustik-Messdaten (A1) von mehreren Stellen der Lunge der Person (P1), wobei das Bereitstellen erst nach Abschluss der Untersuchung, nachdem die Messung der Person (P1) abschlossen ist, beginnt.
Ib. Bereitstellen von Messdaten der elektrischen Impedanz (11) von mehreren Stellen der Lunge der Person (P1), wobei das Bereitstellen erst nach Abschluss der Untersuchung, nachdem die Messung der Person (P1) abschlossen ist, beginnt.
II. Übergabe der Messdaten (A1, 11) an ein trainiertes Künstliches Neuronales Netz (KNN)
III. Vorverarbeiten der Messdaten (A1, 11) in vorverarbeitete Messdaten (A1*, I1*) durch das Künstliche Neuronale Netz (KNN)
IV. Anwendung des trainierten Künstlichen Neuronalen Netzes (KNN) auf die vorverarbeiteten Messdaten (A1*, 11*) zur Bestimmung der Stärke und des räumlichen Ausmaßes der Lungenventilation der Person (P1) durch das KNN, wobei die Werte der Lungenventilation als Ergebnisdaten (D2) erzeugt werden.

2. Verfahren zur Bestimmung der Stärke und des räumlichen Ausmaßes der Lungenventilation gemäß Anspruch 1 **dadurch gekennzeichnet, dass** die Frequenz der Akustik-Messdaten (A1) und der Messdaten der elektrischen Impedanz (11), welche in Schritt Ia und Ib bereitgestellt werden, einen Bereich von 50 bis 3000 Hz umfassen.

3. Verfahren zur Bestimmung der Stärke und des räumlichen Ausmaßes der Lungenventilation gemäß einem der vorangegangenen Ansprüche **dadurch gekennzeichnet, dass** nach Abschluss des Schritts IV aus den Ergebnisdaten (D2) eine dreidimensionale Abbildung erzeugt wird.

4. Vorrichtung zur Durchführung eines Verfahrens zur Bestimmung der Stärke und des räumlichen Ausmaßes der Lungenventilation gemäß einem der vorangegangenen Ansprüche **dadurch gekennzeichnet, dass** es umfasst
• eine Eingabeeinheit (10), die so ausgebildet ist, dass sie Messdaten A1, I1 entweder direkt oder aus einem (Zwischen-) Speicher (90) oder aus einer Datenschnittstelle (50) empfangen und an eine Auswertungseinheit (20) weiterleiten kann,
• eine Auswerteinheit (20) die so ausgebildet ist, dass sie die Messdaten A1, I1 von der Eingabeeinheit (10) oder einer Datenschnittstelle (50) empfangen kann und mittels Künstlichem Neuronalen Netz (KNN) aus den Messdaten A1, I1 sowie aus Trainingsdaten und den daraus enthaltenen erlernten Gewichten die Stärke und das räumliche Ausmaßes der Lungenventilation berechnen kann, so dass Ergebnisdaten (D2) entstehen, die an eine Ausgabeeinheit (30) übermitteln werden,
• eine Ausgabeeinheit (30) die so ausgebildet ist, dass sie die Ergebnisdaten D2 von der Auswertungseinheit (20) empfangen und optisch, akustisch, haptisch und/oder in Form eines Ausdruckes ausgeben kann.

5. Mess-Weste (110) umfassend einen textilen Träger mit einer Vielzahl von Mikrophonen (70) zur Messung von Akustik-Messdaten (A1) sowie einer Vielzahl von Elektroden zur Erzeugung von Messdaten der elektrischen Impedanz (11).
